# EUROPEAN PATENT SPECIFICATION

(11) **EP 2 413 163 B1**
(45) Date of publication and mention of the grant of the patent: **07.10.2015**
(21) Application number: 10174645.1
(22) Date of filing: 31.08.2010
(51) Int. Cl.: G01T 1/29, G01R 33/48, G01R 33/20, G01T 1/16

(54) **Method for removing noise of PET signal using filtering in PET-MRI fusion device and PET system in PET-MRI fusion device using the same**
Verfahren zum Entfernen von Rauschen aus einem PET-Signal mit Filterung in einer PET-MRI-Fusionsvorrichtung und PET-System in der PET-MRi-Fusionsvorrichtung damit
Procédé pour éliminer le bruit d'un signal PET par filtrage dans un dispositif de fusion PET-MRI, système PET dans un dispositif de fusion PET-MRI l'utilisant

(30) Priority: 30.04.2010 KR 20100040513
(43) Date of publication of application: 01.02.2012
(73) Proprietor: Industry-University Cooperation Foundation Sogang University, Seoul (KR)
(72) Inventor: Kang, Jihoon, Jeollanam-do 530-840, (KR); Choi, Yong, Seoul 138-913 (KR); Hong, Key Jo, Seoul 139-939 (KR); Hu, Wei, Seoul 121-809 (KR); Lim, Hyun Keong, Seoul 121-854 (KR); Huh, Yoon Suk, Seoul 140-200 (KR); Kim, Sangsu, Seoul 132-849 (KR)
(74) Representative: Grosse Schumacher Knauer von Hirschhausen

(56) References cited:
- DE-A1-102005 015 070
- HUH Y S ET AL: "Development of filtering methods for PET signals contaminated by RF pulses for combined PET-MRI", 2009 IEEE NUCLEAR SCIENCE SYMPOSIUM AND MEDICAL IMAGING CONFERENCE (NSS/MIC 2009), ORLANDO, FL, USA, IEEE, PISCATAWAY, NJ, USA, 24 October 2009 (2009-10-24), pages 3812-3815, XP031826524, ISBN: 978-1-4244-3961-4

## Description

### CROSS-REFERENCE TO RELATED APPLICATIONS

This application claims priority under 35 U.S.C. §119 to Korean Patent Application No. 10-2010-0040513, filed on April 30, 2010, in the Korean Intellectual Property Office.

### TECHNICAL FIELD

The following disclosure relates to a method for removing noise of a PET signal using filtering in a PET-MRI fusion device, and a PET system in a PET-MRI fusion device using the same.

### BACKGROUND

Medical imaging employed for cellular, pre-clinical and clinical tests and diagnosis of patients is largely classified into structural imaging and functional imaging. Structural imaging refers to structural and anatomical of the human body, and functional imaging refers to imaging of functional information of the cognitive, sensual or other functions of the human body in a direct or indirect manner. The structural or anatomical imaging technique includes computed tomography (CT), magnetic resonance imaging (MRI), or the like. For imaging of functional information about the human body's physiological and biochemical functions, positron emission tomography (PET) is widely used.

PET is a powerful biological imaging tool allowing monitoring of the functional processes of the human body in a noninvasive manner. A biological probe molecule labeled with a radioactive, positron-emitting isotope is injected into the body, and the distribution of radiation is reconstructed through tomography to visualize and quantify the physiological and biochemical responses in the body organs. The functional and molecular biological information about the brain or other organs provided by PET may be useful for the etiological study of disease, diagnosis, prognosis, monitoring after anticancer treatment, or the like.

In order to provide functional information of the human body tissues with a high sensitivity of molecular level and to overcome the problem of low resolution, PET fusion medical imaging devices such as PET-CT, PET-MRI and PET-optical imaging are being developed.

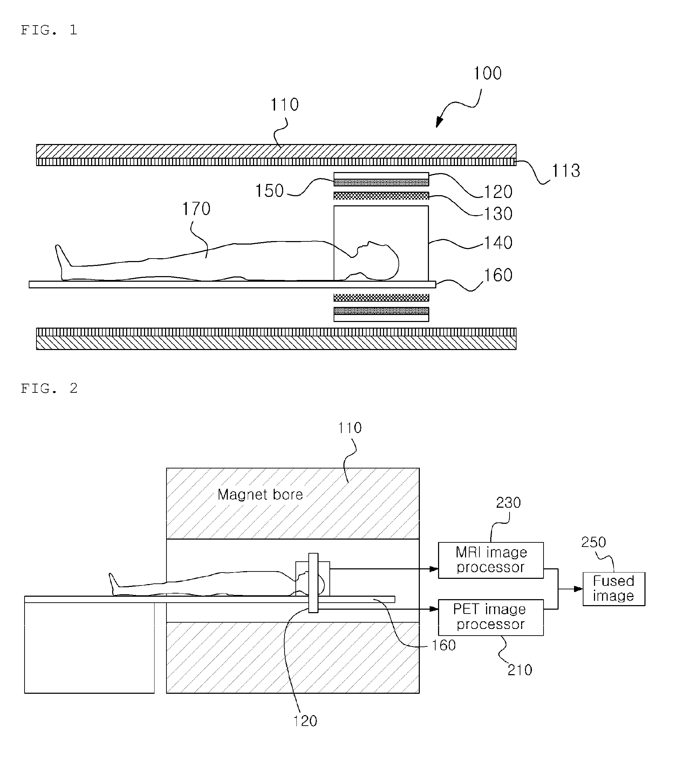

FIG. 1 is a cross-sectional view of an existing PET-MRI fusion device.

Referring to FIG. 1, a PET-MRI fusion device 100 includes a magnet bore 110, a PET detector 120, a radio frequency (RF) receiving coil 130, an RF transmitting coil 140, an RF shield 150 and a bed 160.

The PET detector 120 is provided between the RF receiving coil 130 and the magnet bore 110. The configuration of the PET-MRI fusion device 100 induces interaction between PET and MRI. As a result, various noises that deteriorate image quality are produced. The noises include electromagnetic interference, high-frequency and low-frequency interferences, etc. caused by MRI, and magnetic field distortion,decreasedsignal-to-noise ratio,eddy current, etc. caused by PET. Especially, since the high-magnetic-field, high-frequency energy of MRI has the largest effect on the acquisition of PET signals and the reconstruction of images, the RF shield 150 is provided between the RF receiving coil 130 and the PET detector 120 to minimize the interference. The RF shield 150 may comprise copper (Cu). However, the RF shield 150, which is typically in the form of a conductive cylinder, reduces performance of the RF receiving/transmitting coils 120, 140 of MRI, and degrades resolution of MRI images because of eddy current occurring in a gradient coil 113. Further, since the PET detector 120 is provided outside the RF receiving coil 130 with respect to the center of the magnet bore 110, gamma rays emitted from a subject 170 may be attenuated and diffused by the RF receiving coil 130, thereby resulting in declined PET signal detection ability. Moreover, heating of the PET detector 120 may result in declined performance. Further, the RF shield 150 provided to minimize the high-magnetic-field, high-frequency energy of MRI results in degraded image quality of the PET-MRI fusion device 100.

### SUMMARY

The present disclosure is directed to providing a method for removing noise of a positron emission tomography (PET) signal in a PET-magnetic resonance imaging (MRI) fusion device without using an MRI radio frequency (RF) shield that degrades image quality of the PET-MRI fusion device, and a PET system in a PET-MRI fusion capable of removing noise of a PET signal in a PET-MRI fusion device.

According to the invention, the present disclosure provides a method for removing noise of a PET signal in a PET-MRI fusion device, including: amplifying a PET output signal from a PET-MRI fusion device; converting the amplified PET output signal into a digital signal; and performing digital filtering by removing RF noise based on the relationship between the frequency of the digitized PET output signal and an MR RF frequency (Larmor frequency).

The digital filtering may be performed by removing noise components in the MR RF frequency (Larmor frequency) range and passing the PET output signal only within the frequency range.

In another general aspect, the present disclosure provides a PET system in a PET-MRI fusion device, including: a PET detector detecting gamma rays emitted from a subject and converting flash light changed from the gamma rays into an electrical signal; a signal amplification unit amplifying the electrical signal from the PET detector (PET output signal); a data acquisition unit converting the PET output signal into a digital signal through sampling; and a signal processing unit filtering RF noise based on the relationship between the frequency of the digitized PET output signal and an MR RF frequency (Larmor frequency) and processing the filtered signal for image reconstruction.

The signal processing unit may remove noise components in the MR RF frequency (Larmor frequency) range and pass the PET output signal only within the frequency range.

The method for removing noise of a PET signal using filtering in a PET-MRI fusion device and the PET system in a PET-MRI fusion device according to the disclosure allow acquisition of molecular-level images in MRI environment without declined performance of the PET detector. Decline of performance such as PET non-response time, decreased sensitivity, distortion of images, etc. is minimized. In addition, the method for removing noise of a PET signal using filtering in a PET-MRI fusion device according to the disclosure and the PET system in a PET-MRI fusion device using the same can solve the problem of mutual interference associated with the existing method and system using an RF shield. Further, the presently disclosed system allows easy and simple setup.

Other features and aspects will be apparent from the following detailed description, the drawings, and the claims.

### BRIEF DESCRIPTION OF THE DRAWINGS

The above and other objects, features and advantages of the present disclosure will become apparent from the following description of certain exemplary embodiments given in conjunction with the accompanying drawings, in which:
FIG. 1 is a cross-sectional view of an existing positron emission tomography (PET)-magnetic resonance imaging (MRI) fusion device;
FIG. 2 illustrates a PET-MRI fusion device according to an embodiment;
FIG. 3 is a graph showing a normal PET pulse signal of a PET detector;
FIG. 4 is a graph showing a PET pulse signal affected by noise of high-magnetic-field, high-frequency energy of MRI in a PET-MRI fusion device;
FIG. 5 is a flow chart illustrating an exemplary method for removing noise of a PET signal using filtering in a PET-MRI fusion device ;
FIG. 6 is a flow chart illustrating a method for removing noise of a PET signal using filtering in a PET-MRI fusion device according to another embodiment;
FIG. 7 is a block diagram illustrating a PET system in a PET-MRI fusion device according to an example;
FIG. 8 is a circuit diagram illustrating a noise filter unit according to an embodiment;
FIG. 9 is a circuit diagram illustrating a noise filter unit according to another embodiment;
FIG. 10 is a block diagram illustrating a PET system in a PET-MRI fusion device according to another embodiment; and
FIG. 11 is a graph showing an energy spectrum after removal of noise of a PET signal in a PET-MRI fusion device according to an embodiment.

It should be understood that the appended drawings are not necessarily to scale, presenting a somewhat simplified representation of various preferred features illustrative of the basic principles of the disclosure. The specific design features of the disclosure as disclosed herein, including, for example, specific dimensions, orientations, locations and shapes, will be determined in part by the particular intended application and use environment.

In the figures, reference numerals refer to the same or equivalent parts of the disclosure throughout the several figures of the drawings.

### DETAILED DESCRIPTION OF EMBODIMENTS

Hereinafter, description will be made about particular embodiments of the present disclosure which may be variously modified. However, it is not intended to limit the present disclosure to specific embodiments. On the contrary, the present disclosure is intended to cover not only the exemplary equivalents but also various alternatives, modifications, equivalents and other equivalents that may be included within the spirit and scope the present disclosure are.

While terms including ordinal numbers, such as "first", "second", etc., may be used to describe various components, such components are not limited to the above terms. Those terms are used only to distinguish one component from another. For example, without departing from the scope of the present disclosure, a first component may be referred to as a second component, and likewise a second component may be referred to as a first component. The term and/or encompasses both combinations of the plurality of related items disclosed and any one item from among the plurality of related items disclosed.

When a component is mentioned to be "connected" to or "accessing" another component, this may mean that it is directly connected to or accessing the other component, but it is to be understood that another component may exist in between. On the other end, when a component is mentioned to be "directly connected" to or "directly accessing" another component, it is to be understood that there are no other components in between.

The terms used in the present specification are merely used to describe particular embodiments, and are not intended to limit the present disclosure. An expression used in the singular encompasses the expression of the plural, unless it has a clearly different meaning in the context. In the present specification, it is to be understood that the terms such as "including", "having", etc. are intended to indicate the existence of the features, numbers, operations, components, parts or combinations thereof disclosed in the specification, and are not intended to preclude the possibility that one or more other features, numbers, operations, components, parts or combinations thereof may exist or may be added.

Unless otherwise defined, all terms used herein, including technical and scientific terms, have the same meanings as those generally understood by those with ordinary knowledge in the field of art to which the present disclosure belongs. Such terms as those defined in a generally used dictionary are to be interpreted to have the meanings equal to the contextual meanings in the relevant filed of art, and are not to be interpreted to have ideal or excessively formal meanings unless clearly defined in the present specification.

Certain embodiments of the present disclosure will be described below in more detail with reference to the accompanying drawings, in which those components are rendered the same reference numeral that are the same or are in correspondence, regardless of the figure number, and redundant explanations are omitted.

FIG. 2 illustrates a positron emission tomography (PET)-magnetic resonance imaging (MRI) fusion device according to an embodiment.

Referring to FIG. 2, a PET system comprising a PET detector 120 and a PET image processor 210 removes noise of a PET signal in a PET-MRI fusion device. The MRI operates independently.

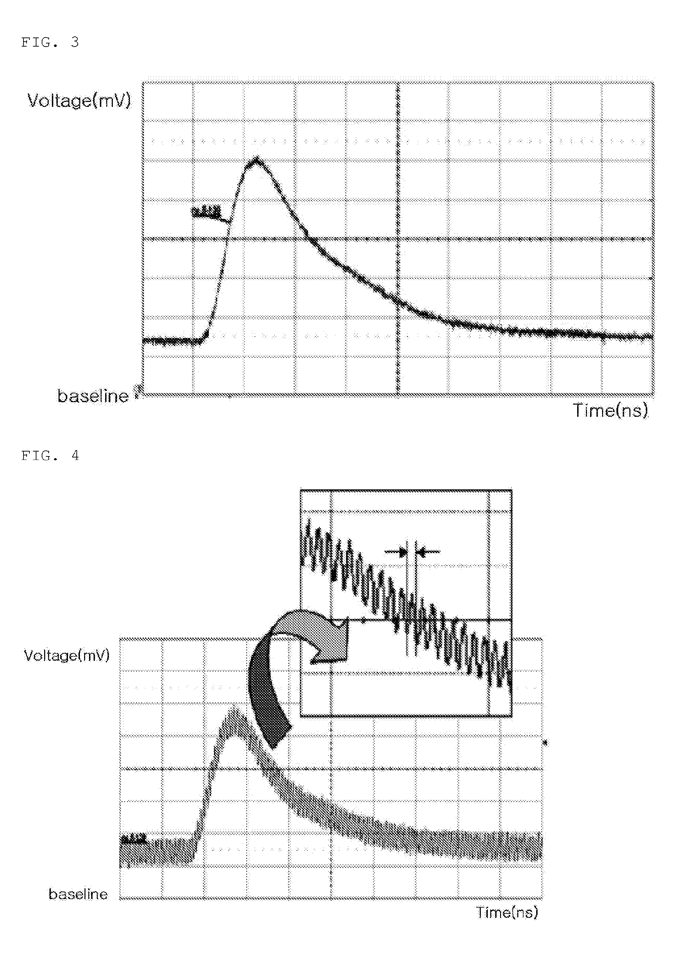

FIG. 3 is a graph showing a normal PET pulse signal of a PET detector.

FIG. 4 is a graph showing a PET detector pulse signal affected by noise of high-magnetic-field, high-frequency energy of MRI in a PET-MRI fusion device. Referring to FIG. 4, the signal is not clear because of noise.

In a method for removing noise of a PET signal in a PET-MRI fusion device, magnetic resonance (MR) radio frequency (RF) noise is filtered based on the relationship between the MR RF frequency and the frequency of a PET output signal, thereby removing the noise of the PET signal.

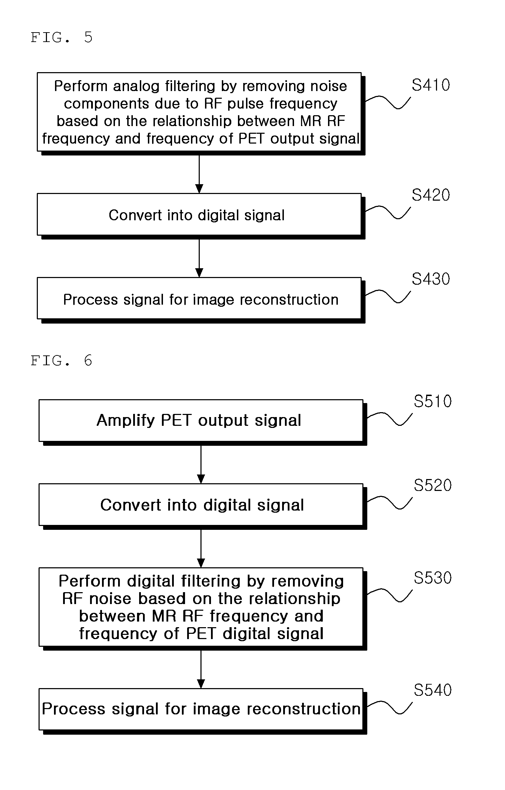

FIG. 5 is a flow chart illustrating a method for removing noise of a PET signal using filtering in a PET-MRI fusion device according to an example.

Referring to FIG. 5, a PET output signal is input from a PET-MRI fusion device and subjected to analog filtering by removing noise components due to an RF pulse frequency based on the relationship between the frequency of the PET output signal and an MR RF frequency (Larmor frequency) (S410). That is to say, the PET output signal only within the frequency range is passed. Through this, RF noise absorbed by a PET signaling cable or the signal amplification unit is removed by filtering. For example, if a PET output signal with a frequency of 100 MHz (whose main frequency component is in the 100 MHz range) or lower is affected by RF noise in the 300 MHz range, the RF noise in the 300 MHz range may be filtered by designing a low-pass filter for 100 MHz or lower and a band-rejection filter for the 100 MHz range and the 300 MHz range. An analog filter circuit may be an RLC filter circuit consisting of passive elements or an active filter circuit consisting of active elements. The analog filter circuit may be provided in an MR bore or inside or on the wall of an MR scanning room.

Then, the filtered signal is converted into a digital signal through sampling (S420), and then processed for image reconstruction (S430) .

FIG. 6 is a flow chart illustrating a method for removing noise of a PET signal using filtering in a PET-MRI fusion device according to the invention.

Referring to FIG. 6, a PET output signal is received and then amplified (S510). Then, the amplified PET output signal is converted into a digital signal (S520). Subsequently, digital filtering is performed by removing RF noise based on the relationship between the frequency of the digitized PET output signal and an MR RF frequency (Larmor frequency) (S530). Then, the filtered signal is processed for image reconstruction (S540). The digital filtering may be performed by removing noise components in the MR RF frequency (Larmor frequency) range and passing the PET output signal only within the frequency range. The digital filtering may be performed using a logic circuitry such as a field-programmable gate array (FPGA), a complex programmable logic device (CPLD), etc. A digital filter may be provided inside or outside the MR scanning room.

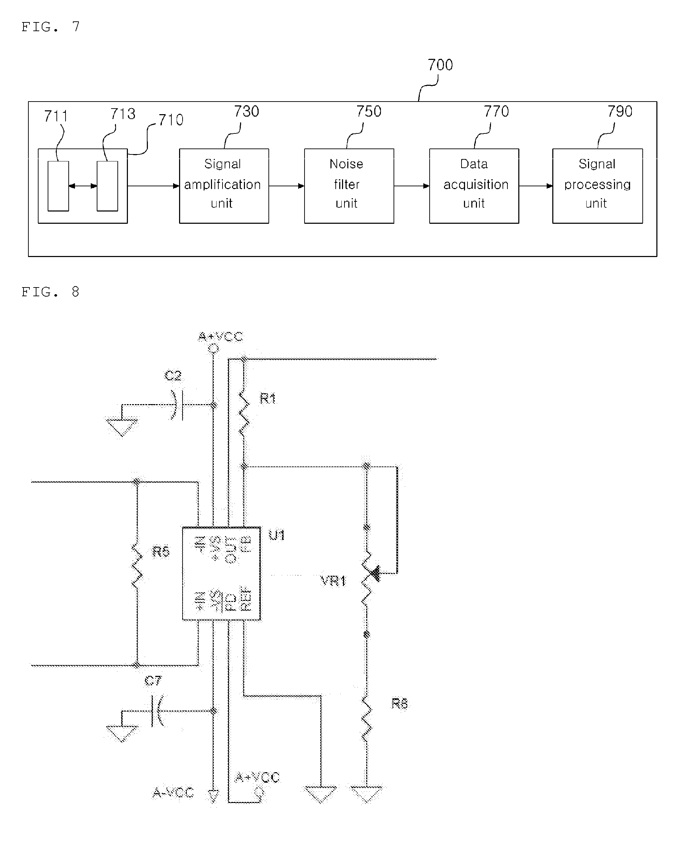

FIG. 7 is a block diagram illustrating a PET system in a PET-MRI fusion device according to an example.

Referring to FIG. 7, a PET system 700 in a PET-MRI fusion device comprises a PET detector 710, a signal amplification unit 730, a noise filter unit 750, a data acquisition unit 770 and a signal processing unit 790.

The PET detector 710 comprises a scintillation crystal 711 and a photosensor 713. The scintillation crystal 711 detects gamma rays emitted from a subject and converts them into a flash light. For example, the scintillation crystal 711 detects 511 keV gamma rays emitted through a pair annihilation phenomenon toward opposite directions. The scintillation crystal 711 may be selected from bismuth germanate (BGO), lutetium oxyorthosilicate (LSO), lutetium yttrium oxyorthosilicate (LYSO), lutetium aluminum perovskite (LuAP), lutetium yttrium aluminum perovskite (LuYAP), lanthanum bromide (LaBr₃), lutetium iodide (LuI₃), gadolinium oxyorthosilicate (GSO), lutetium gadolinium oxyorthosilicate (LGSO) and lutetium aluminum garnet (LuAG). The photosensor 713 may be a photomultiplier tube (PMT), a positive-intrinsic-negative PIN) diode, cadmium telluride (CdTe), cadmium zinc telluride (CZT), an avalanche photodiode (APD), a Geiger-mode avalanche photodiode (GAPD), or the like.

The signal amplification unit 730 amplifies weak electrical signals input from the PET detector 710 and increases number of signal channels to enable data acquisition and signal processing. The noise filter unit 750 filters RF noise based on the relationship between the frequency of the PET output signal and an MR RF frequency (Larmor frequency). That is to say, it removes noise components in the MR RF frequency (Larmor frequency) range and passes the PET output signal only within the frequency range. Those skilled in the art will understand that the noise filter unit 750 may be configured by a combination of various filters including a high-pass filter, a low-pass filter, a band-pass filter and a band-rejection filter.

The data acquisition unit 770 converts the RF noise-filtered electrical signal (hereinafter, referred to as a PET signal) into a digital signal through sampling to allow signal processing and image reconstruction. The signal processing unit 790 processes the resulting PET signal for image reconstruction.

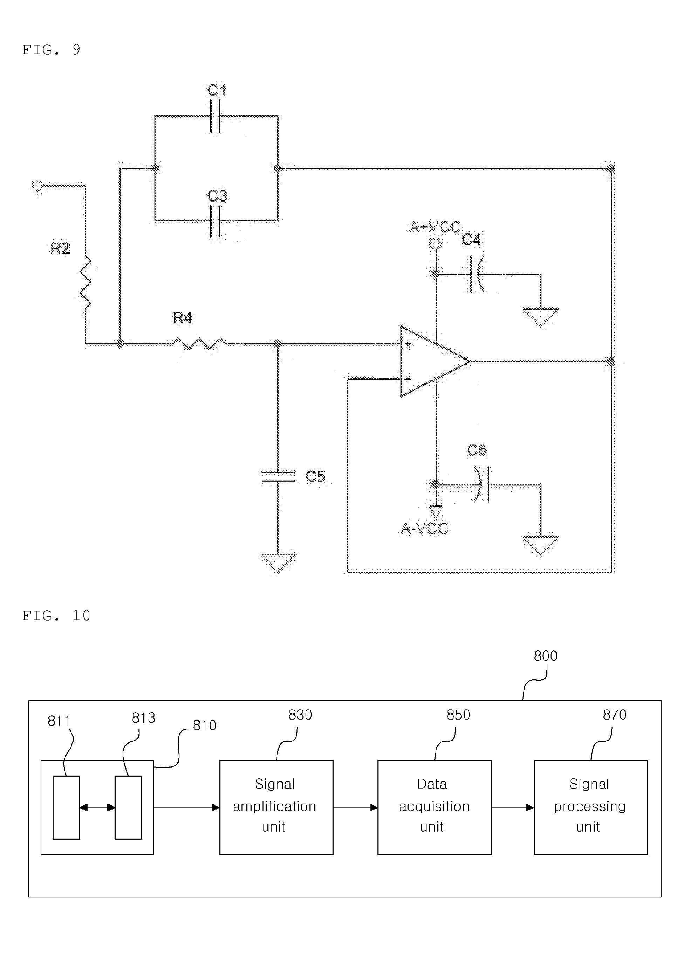

FIG. 8 is a circuit diagram illustrating a noise filter unit according to an embodiment, and FIG. 9 is a circuit diagram illustrating a noise filter unit according to another embodiment. Those skilled in the art will understand that the noise filter unit may be configured variously depending on the relationship between an MR RF frequency (Larmor frequency) and the frequency of the PET output signal

FIG. 10 is a block diagram illustrating a PET system in a PET-MRI fusion device according to another embodiment.

Referring to FIG. 10, a PET system 800 in a PET-MRI fusion device comprises a PET detector 810, a signal amplification unit 830, a data acquisition unit 850 and a signal processing unit 870.

The PET detector 810 comprises a scintillation crystal 811 and a photosensor 813. It detects gamma rays emitted from a subject and converts flash light changed from the gamma rays into an electrical signal. The signal amplification unit 830 amplifies weak electrical signals input from the PET detector 810 and increases number of signal channels to enable data acquisition and signal processing.

The data acquisition unit 850 converts the electrical analog signal into a digital signal through sampling. The signal processing unit 870 filters RF noise based on the relationship between the frequency of the PET output signal and an MR RF frequency (Larmor frequency). That is to say, it removes noise components in the MR RF frequency (Larmor frequency) range and passes the PET output signal only within the frequency range. For example, a finite impulse response (FIR) filter, an infinite impulse response (IIR) filter, or the like may be used. The signal processing unit 870 processes the filtered signal for image reconstruction.

A method for removing noise of a PET signal using filtering in a PET-MRI fusion device according to an embodiment of the disclosure may be used at the same time with a method for removing noise of a PET signal in a PET-MRI fusion device according to another embodiment of the disclosure. And, the feature of a PET system in a PET-MRI fusion device according to an embodiment of the disclosure may exist together with the feature of a PET system in a PET-MRI fusion device according to another embodiment of the disclosure.

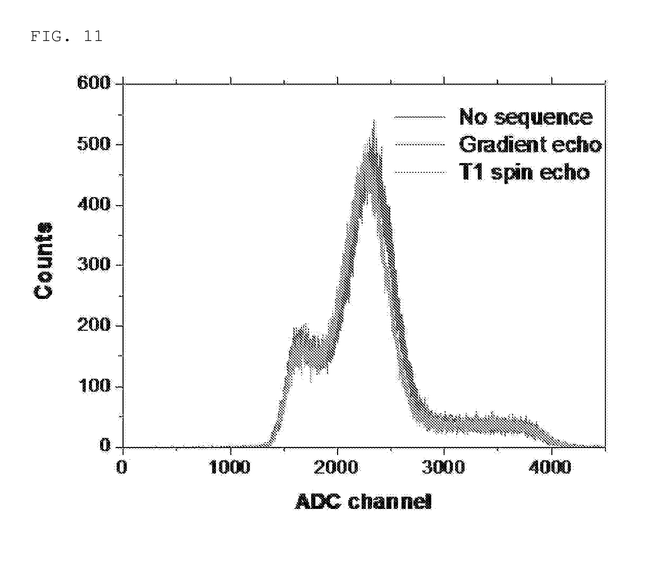

FIG. 11 is a graph showing an energy spectrum after removal of noise of a PET signal in a PET-MRI fusion device according to an embodiment. Referring to FIG. 11, it can be seen that the noise was removed.

The term "unit" used in the specification refers to, but is not limited to, a software or hardware component, such as an FPGA or an application-specific integrated circuit (ASIC), which executes certain tasks. A unit may be configured to reside in the addressable storage medium, and configured to execute on one or more processors. Thus, a unit may include, by way of example, components, such as software components, object-oriented software components, class components and task components, processes, functions, attributes, procedures, subroutines, segments of program code, drivers, firmware, microcode, circuitry, data, databases, databases structures, tables, arrays and variables. The functionality provided for in the components and units may be combined into fewer components and units or further separated into additional components and units. In addition, the components and units may be implemented such that they execute one or more CPU (s) in a device or a secure multimedia card.

The specific numbers used in the above embodiments are given only to describe the embodiments of the present disclosure, and the present disclosure is not limited by those specific numbers.

The functionalities described above may be implemented by a processor such as a microprocessor, a controller, a microcontroller, an ASIC, etc. according to software or program codes coded to execute such functionalities. Designing, development and implementation of such codes will be easily understood by those skilled in the art based on the description of the present disclosure.

For example, the program may be recorded on a hard disk or in a read-only memory (ROM) as a recording medium in advance. Alternatively, the program may be temporarily or permanently stored on a removable recording medium such as a flexible disk, a compact disc read-only memory (CD-ROM), a magneto-optical (MO) disk, a digital versatile disc (DVD), a magnetic disk or a semiconductor memory. Such a removable recording medium can be provided as package software.

## Claims

1. A method for removing noise of a positron emission tomography (PET) signal in a PET-magnetic resonance imaging (MRI) fusion device, comprising:
amplifying a PET output signal from a PET-MRI fusion device (S510);
converting the amplified PET output signal into a digital signal (S520); and
**characterized in that** the method further comprises
performing digital filtering by removing radio frequency (RF) noise based on the relationship between the frequency of the digitized PET output signal and a magnetic resonance (MR) RF frequency (Larmor frequency) (S530).

2. The method for removing noise of a PET signal in a PET-MRI fusion device according to claim 1, wherein said digital filtering comprises removing noise components in the MR RF frequency (Larmor frequency) range and passing the PET output signal whose main frequency component is in the 100 MHz range only within the frequency range.

3. A positron emission tomography (PET) -magnetic resonance imaging (MRI) fusion device, comprising:
a PET detector (810) adapted to detect gamma rays emitted from a subject and converting flash light changed from the gamma rays into an electrical signal;
a signal amplification unit (830) adapted to amplify the electrical signal from the PET detector (810) (PET output signal);
a data acquisition unit (850) adapted to convert the PET output signal into a digital signal through sampling; and
**characterized by** a signal processing unit (870) adapted to filter radio frequency (RF) noise based on the relationship between the frequency of the digitized PET output signal and a magnetic resonance (MR) RF frequency (Larmor frequency) and the signal processing unit further adapted to process the filtered signal for image reconstruction.

4. The PET-MRI fusion device according to claim 3, wherein the signal processing unit (870) is adapted to remove noise components in the MR RF frequency (Larmor frequency) range and to pass the PET output signal whose main frequency component is in the 100 MHz range only within the frequency range.

## Patentansprüche

1. Verfahren zum Entfernen von Rauschen eines Positronen-Emissions-Tomographie-Signals (PET-Signals) in einer PET-Magnetresonanztomographie-Verknüpfungsvorrichtung (PET-MRI-Verknüpfungsvorrichtung) aufweisend:
Verstärken eines PET-Ausgabesignals von einer PET-MRI-Verknüpfungsvorrichtung (S510);
Umwandeln des verstärkten PET-Ausgabesignals in ein digitales Signal (S520); und
**dadurch gekennzeichnet, dass** das Verfahren weiter umfasst
Durchführen eines digitalen Filterns durch Entfernen von Hochfrequenzrauschen (RF = Hochfrequenz), basierend auf der Beziehung zwischen der Frequenz des digitalisierten PET-Ausgabesignals und einer hochfrequenten Magnetresonanzfrequenz (MR-RF-Frequenz) (Larmor-Frequenz) (S530).

2. Verfahren zum Entfernen von Rauschen aus einem PET-Signal in einer PET-MRI-Verknüpfungsvorrichtung gemäß Anspruch 1, wobei das digitale Filtern beinhaltet, dass Rauschkomponenten im MR-RF-Frequenzbereich (Larmor-Frequenzbereich) entfernt werden und das PET-Ausgabesignal, dessen Hauptfrequenzkomponente im 100 MHz-Bereich liegt, lediglich innerhalb des Frequenzbereiches durchgelassen wird.

3. Positronenemissionstomographie-Magnetresonanztomographie-Verknüpfungsvorrichtung (PET-MRI-Verknüpfungsvorrichtung) aufweisend:
einen PET-Detektor (810), der ausgebildet ist, um von einem Gegenstand ausgesendete Gammastrahlen zu erfassen und aus den Gammastrahlen umgewandeltes Blitzlicht in ein elektrisches Signal umzuwandeln;
eine Signalverstärkereinheit (830), die ausgebildet ist, das elektrische Signal von dem PET-Detektor (810) (PET-Ausgabesignal) zu verstärken;
eine Datenbeschaffungseinheit (850), die ausgebildet ist, das PET-Ausgabesignal durch Abtastung (Sampling) in ein digitales Signal umzuwandeln; und
**gekennzeichnet durch** eine Signalverarbeitungseinheit (870), die ausgebildet ist, um Hochfrequenzrauschen (RF = Hochfrequenz) zu filtern, und zwar basierend auf der Beziehung zwischen der Frequenz des digitalisierten PET-Ausgabesignals und einer hochfrequenten Magnetresonanzfrequenz (MR-RF-Frequenz) (Larmor-Frequenz), und die Signalverarbeitungseinheit weiter ausgebildet ist, um das gefilterte Signal für eine Bildrekonstruktion zu verarbeiten.

4. PET-MRI-Verknüpfungsvorrichtung nach Anspruch 3, wobei die Signalverarbeitungseinheit (870) ausgebildet ist, um Rauschkomponenten im MR-RF-Frequenzbereich (Larmor-Frequenzbereich) zu entfernen und das PET-Ausgabesignal, dessen Hauptfrequenzkomponente im 100 MHz-Bereich liegt, lediglich innerhalb des Frequenzbereiches durchzulassen.

## Revendications

1. Procédé pour supprimer le bruit d'un signal de tomographie par émission de positons (TEP) dans un dispositif de fusion TEP-imagerie par résonance magnétique (IRM), comprenant :
l'amplification d'un signal de sortie TEP en provenance d'un dispositif de fusion TEP-IRM (S510) ;
la conversion du signal de sortie TEP amplifié en un signal numérique (S520) ; et
**caractérisé en ce que** le procédé comprend en outre
l'exécution d'un filtrage numérique en supprimant le bruit radiofréquence (RF) sur la base de la relation entre la fréquence du signal de sortie TEP numérisé et une fréquence RF de résonance magnétique (RM) (fréquence de Larmor) (S530).

2. Procédé pour supprimer le bruit d'un signal TEP dans un dispositif de fusion TEP-IRM selon la revendication 1, dans lequel ledit filtrage numérique comprend la suppression de composantes de bruit dans la gamme de fréquences RF RM (fréquence de Larmor) et le passage du signal de sortie TEP dont la composante fréquentielle principale est dans la gamme de 100 MHz uniquement à l'intérieur de la gamme de fréquences.

3. Dispositif de fusion tomographie par émission de positons (TEP) - imagerie par résonance magnétique (IRM), comprenant :
un détecteur TEP (810) adapté pour détecter des rayons gamma émis depuis un sujet et convertir la lumière flash changée à partir des rayons gamma en un signal électrique ;
une unité d'amplification de signal (830) adaptée pour amplifier le signal électrique en provenance du détecteur TEP (810) (signal de sortie TEP) ;
une unité d'acquisition de données (850) adaptée pour convertir le signal de sortie TEP en un signal numérique par le biais d'un échantillonnage ; et
**caractérisé par** une unité de traitement de signal (870) adaptée pour filtrer le bruit radiofréquence (RF) sur la base de la relation entre la fréquence du signal de sortie TEP numérisé et une fréquence RF de résonance magnétique (RM) (fréquence de Larmor) et l'unité de traitement de signal en outre adaptée pour traiter le signal filtré pour la reconstruction d'image.

4. Dispositif de fusion TEP-IRM selon la revendication 3, dans lequel l'unité de traitement de signal (870) est adaptée pour supprimer des composantes de bruit dans la gamme de fréquences RF RM (fréquence de Larmor) et pour passer le signal de sortie TEP dont la composante fréquentielle principale est dans la gamme de 100 MHz uniquement à l'intérieur de la gamme de fréquences.
